# EUROPEAN PATENT APPLICATION

(11) **EP 0 654 531 A1**
(43) Date of publication of application: **24.05.1995**
(21) Application number: 94203083.4
(22) Date of filing: 24.10.1989
(51) Int. Cl.: C12N 15/54, C12N 15/82, C12Q 1/68, A01H 5/00, C12N 9/12

(54) **ADP glucose-pyrophosphorylase**

(30) Priority: 10.11.1988 GB 8826356
(62) Divisional of application: 89310973.6
(71) Applicant: ZENECA LIMITED, London W1Y 6LN (GB)
(72) Inventor: Bridges, Ian George, Hampshire RG7 2LL (GB); Olive, Mark, Cook, Act 2614 (AU); Schuch, Wolfgang Walter, Crowthorne, Berkshire (GB)
(74) Representative: Huskisson, Frank Mackie

(57) **Abstract**

The starch-synthesising ability of plants, particularly cereals, may be modified by insertion into their genomes by genetic transformation a DNA encoding ADP-glucose pyrophosphorylase. Extra copies of the DNA enhance and antisense variants thereof reduce the starch-synthesising ability of the plants by enhancing or inhibiting expression of the enzyme.

## Description

This invention relates to the isolation, purification and characterisation of the enzyme ADP-glucose pyrophosphorylase and the production of antibodies which can be used in the the identification of ADP-glucose pyrophosphorylase cDNA clones. The invention also relates to the use of cDNA clones to increase starch yield in cereals such as wheat, maize and barley is described.

Starch is an important end-product of carbon fixation during photosynthesis in leaves and is an important storage product in seeds and fruits. In economic terms, the starch produced by the edible portions of three grain crops, wheat, rice and maize, provide approximately two-thirds of the world's food calculated as calories.

Starch is synthesised in the plastid compartment, the chloroplast, in photosynthetic cells or the amyloplast in non- photosynthetic cells. The biochemical pathway of starch biosynthesis in leaves has been well-characterised. In contrast, little is known of the pathway of starch biosynthesis in storage organs. However, the recent development of a method for the isolation of intact amyloplasts from wheat endosperm cells has contributed to the elucidation of the pathway of starch biosynthesis in that organ. Other research has shown that the pathways of starch biosynthesis in wheat endosperm and wheat leaves are different.

The plastid enzyme ADP-glucose pyrophosphorylase is an important regulatory enzyme of starch biosynthesis in photosynthetic plant organs. The chloroplast ADP-glucose pyrophosphorylase is regulated post-translationally by the allosteric effectors 3-phosphoglycerate and orthophosphate. All plant leaf ADP-glucose pyrophosphorylase enzymes studied are activated in vitro by 3-phosphoglycerate, and to a lesser extent by fructose-1,6-bisphosphate, fructose-6-phosphate and phosphoenol pyruvate. These metabolites lower the Km values of substrates and increase the Vₘₐₓ of the enzyme-catalysed reaction.

In addition, the ADP-glucose pyrophosphorylase enzymes from plant leaves are inhibited in vitro by orthophosphate, with half-maximal inhibition of enzyme activity generally achieved in orthophosphate concentrations of less than µ100 M.

Leaf starch biosynthesis is regulated in vitro by fluctuations in the chloroplastic levels of 3-phosphoglycerate and orthophosphate, at the level of the ADP-glucose pyrophosphorylase enzyme. Crucial to this regulatory mechanism is the selective permeability of the chloroplast inner membrane. It has been shown that the inner membrane of chloroplasts is selectively permeable to triose-phosphates, dicarboxylates and orthophosphate, which are rapidly and specifically transported from the chloroplast to the cytosol and vice versa. This active transport mechanism involves the counter-balancing inward movement of orthophosphate from the cytosol via the phosphate/triose-phosphate translocator. The active transport of metabolites via the orthophosphate/ triose-phosphate translocator is involved in altering the ratio of orthophosphate to 3-phosphoglycerate (i.e. [Pi]/[3-PGA]) within the plastid during normal light-dark transitions. During photosynthesis, the 3-phosphoglycerate formed from CO₂ -fixation accumulates in the chloroplast. The concentration gradient produced in 3-phosphoglycerate from the inside of the chloroplast to the cytosol leads to the export of some 3-phosphoglycerate into the cytosol in exchange for orthophosphate. The imported orthophosphate is subsequently utilised in ATP formation via photophosphorylation, so that the overall chloroplastic ratio of [Pi]/[3-PGA] remains low during the light cycle. Thus the ADP-glucose pyrophosphorylase enzyme is allosterically activated in the light, allowing starch biosynthesis to continue. During the dark cycle, there is decreased CO₂ -fixation and decreased photophosphorylation, coupled with increased chloroplastic orthophosphate concentration as a result of the hydrolysis of ATP. This produces a high chloroplastic ratio of [Pi]/[3-PGA], thereby inhibiting ADP-glucose pyrophosphorylase activity and starch formation.

In addition to the modulation of ADP-glucose pyrophosphorylase activity by allosteric effectors, leaf starch biosynthesis is also probably regulated by the adenylate energy charge of the chloroplast.

The most important regulatory feature of starch biosynthesis in the developing endosperm is probably a coarse control mechanism via regulation of the synthesis of starch biosynthetic enzymes. There has been considerable emphasis in recent research on the identification of a rate-limiting enzyme in the starch biosynthetic pathway. Unfortunately, studies measuring the in vitro enzyme activities of starch biosynthetic enzymes throughout cereal endosperm development have not shed much light on this problem, since it is difficult to correlate in vitro and in vivo enzyme activities and most of the starch biosynthetic enzymes appear to be expressed co-ordinately.

At present there is no substantial evidence that post- translational regulation of ADP-glucose pyrophosphorylase operates in vivo in cereal storage organs. The amyloplasts of cereal grains do not develop into chloroplasts during normal development and are functionally unrelated. This factor, coupled with the lack of involvement of triose phosphates in the starch biosynthetic pathway in wheat endosperm suggests that there would be no role in vivo for allosteric regulation of ADP-glucose pyrophosphorylase in cereal endosperm amyloplasts. However, the ADP-glucose pyrophosphorylase enzyme from maize endosperm is activated in vitro by 3-phospho- glycerate to a similar extent as the enzyme isolated from plant leaves. In contrast, the ADP-glucose pyrophosphorylase enzyme from rice endosperm is only activated 1.2-fold in vitro, by 5 mM 3-phosphoglycerate.

For all cereal endosperm enzymes, the presence of 3-phospho- glycerate reduces the sensitivity of the enzyme to inhibition by orthophosphate, indicating that 3-phosphoglycerate binds to the enzyme, close to the site for binding of orthophosphate. Thus, despite the conservation of sites for the binding of allosteric effectors in the endosperm ADP-glucose pyrophosphorylase enzymes, and the ability of the maize endosperm enzyme to be strongly activated in vitro by 3-phosphoglycerate, the in vivo significance of these allosteric effects is uncertain.

An object of the present invention is to provide ADP-glucose pyrophosphorylase in substantially pure form.

According to the present invention there is provided a plasmid designated WL:AGA.1 containing a cDNA encoding ADP-glucose pyrophosphorylase which plasmid has been deposited in an E.coli strain TG2 host with the National Collection of Industrial & Marine Bacteria, Aberdeen, U.K. on 19th October 1988: Accession No. NCIB 40065.

The invention also provides a cDNA encoding ADP-glucose pyrophosphorylase and having the same nucleotide sequence as that of the insert in plasmid WL:AGA.1 or a sequence which is sufficiently similar thereto to to be capable of expressing a protein possessing ADP-glucose pyrophosphorylase activity.

Further according to the invention there is provided a plasmid designated WE:AGA.3 containing a cDNA encoding ADP-glucose pyrophosphorylase which plasmid has been deposited in an E.coli strain TG2 host with the National Collection of Industrial & Marine Bacteria, Aberdeen, U.K. on 19th October 1988: Accession No. NCIB 40066.

The invention also provides a cDNA encoding ADP-glucose pyrophosphorylase and having the same nucleotide sequence as that of the insert in plasmid WL:AGA.3 or a sequence which is sufficiently similar thereto as to be capable of expressing a protein possessing ADP-glucose pyrophosphorylase activity.

Still further according to the invention there is provided a plasmid designated WE:AGA.7 containing a cDNA encoding ADP-glucose pyrophosphorylase which plasmid has been deposited in an E.coli strain TG2 host with the National Collection of Industrial & Marine Bacteria, Aberdeen, U.K. on 19th October 1988: Accession No. NCIB 40067.

Also, the invention provides a cDNA encoding ADP-glucose pyrophosphorylase and having the same nucleotide sequence as that of the insert in plasmid WL:AGA.7 or a sequence which is sufficiently similar thereto to to be capable of expressing a protein possessing ADP-glucose pyrophosphorylase activity.

These clones may be used as probes for equivalent genes in other plant species particularly cereals such as wheat, maize, barley and sorghum and other food crops such as potatoes. Therefore, the present invention, by providing the aforesaid cDNAs, also provides a method of detecting the presence of a DNA sequence encoding ADP-glucose pyrophosphorylase comprising detecting a DNA digest with a gene probe comprising a cDNA aforesaid.

Furthermore the invention provides a plant having enhanced ability to produce starch comprising a starch-synthesising plant having stably incorporated within its genome by transformation one or more than one additional copy of a gene encoding ADP-glucose pyrophosphorylase.

Additionally the invention provides a plant having reduced ability to produce starch comprising a starch-synthesising plant having stably incorporated within its genome by transformation a gene encoding a mRNA antisense to the mRNA encoded by the endogenous ADP-glucose pyrophosphorylase gene of the plant.

The principal utility of the cDNAs defined is in the transformation of crop plants to regulate starch biosynthesis and, accordingly, the invention also provides transformed plants containing one or more copies of one or more of the cDNAs (i), (ii) and (iii) defined above.

The description which follows will describe a method for the isolation of ADP-glucose pyrophosphorylase from wheat, the kinetic properties of the wheat endosperm and leaf enzymes, methods for the isolation of cDNA clones encoding ADP-glucose pyrophosphorylase. There will also be described in detail the structural features of these cDNA clones. These clones can be used for the isolation of the corresponding genes. Both the cDNAs and the genes can then be used in studies leading to the increase in starch yield. One possible application could be the use of these sequences to increase gene dosage of ADP-glucose pyrophosphorylase in transformed crop plants to determine the contribution of ADP-glucose pyrophosphorylase to the net regulation of starch biosynthesis, and perhaps to subsequently modify the levels of starch accumulated during for example grain filling in cereals. The introduction of additional copies of wheat endosperm ADP-glucose pyrophosphorylase genes should produce greater levels of the enzyme in wheat endosperm amyloplasts. Increased gene expression may also be elicited by introducing multiple copies of enhancer sequences into the 5'-untranscribed region of ADP-glucose pyrophosphorylase gene. If the enzyme is rate-limiting to starch biosynthesis, then the rate of starch biosynthesis would be expected to increase in the transformed plants. By virtue of this invention it will also be possible to alter the kinetic properties of the endopserm enzyme through protein engineering, e.g. by the manipulation of the orthophosphate binding site to make the enzyme less sensitive to orthophosphate inhibition. Obviously a number of other parameters could also be improved. The present invention will now be described, by way of illustration, by the following Example.

The reactions involved in the biosynthetic pathways of starch and glucose in leaves are catalysed by the following enzymes:
1) phosphoglycerate kinase/glyceraldehyde-3-phosphate dehydrogenase
2) triose-phosphate isomerase
3) aldolase
4) fructose-1,6-bisphosphatase
5) hexose phosphate isomerase
6) phosphoglucomutase
7) ADP-glucose pyrophosphorylase
8) starch synthase
9) UDP-glucose pyrophosphorylase
10) sucrose phophate synthase
11) sucrose phosphatase
12) orthophosphate/triose phosphate translocator
13) inorganic pyrophosphatase
In wheat endosperm the proposed metabolic pathway of starch biosynthesis the enzymes involved are: (Keeling et. al. 1988).
1) sucrose synthase
2) UDP-glucose pyrophosphorylase
3) hexokinase
4) phosphoglucomutase
5) hexose-phosphate isomerase
6) ATP-dependent phosphofructokinase
7) PPᵢ-dependent phosphofructokinase
8) aldolase
9) triose-phosphate isomerase
10) hexose-phosphate translocator (?)
11) ADP-glucose pyrophosphorylase
12) starch synthase
13) sucrose phosphate synthase
14) sucrose phophatase

### 1.IDENTIFICATION AND PURIFICATION OF WHEAT ENDOSPERM ADP-GLUCOSE PYROPHOSPHORYLASE

### 1.1 Purification of the enzyme

ADP-glucose pyrophosphorylase was partially purified by chromatography on phenyl-Sepharose, followed by two cycles of purification on the ion-exchange medium MonoQ HR 5/5 and one cycle of purification through the gel filtration medium, Superose 12 HR 1-/30. Typical purification results are presented in. Pooled ADP-glucose pyrophosphorylase extracts at each stage of the purification procedure were analysed on an SDS/polyacrylamide gel. Following gel filtration there were very few protein contaminants in the enzyme preparation, which appeared to be greater than 90% pure. A polypeptide of 51,000 molecular weight was selectively purified at each stage of the purification. Thus, the wheat endosperm ADP-glucose pyrophosphorylase enzyme has a subunit molecular weight of 51,000.

The native molecular weight of wheat endosperm ADP-glucose pyrophosphorylase was determined to be 245,000 ± 30,000, by re-chromatography of the partially purified enzyme on Superose 12 HR 10/30. This is consistent with the estimated molecular weight of 260,000 ± 20,000, determined by chromatography of the protein on Sephacryl S-300 sf. In each case, the molecular weight was determined by comparison of the elution volume of ADP-glucose pyrophosphorylase activity with the elution volume of known protein standards, ferritin, catalase and bovine serum albumin. Thus, we conclude, the wheat endosperm enzyme consists of four subunits of identical molecular weight.

### 1.2 Kinetic properties of the enzyme

Partially-purified wheat endosperm ADP-glucose pyro- phosphorylase is not activated by concentrations of up to 20 mM 3-phosphoglycerate. In contrast, the wheat leaf enzyme is activated 14.5-fold by 100 µM 3-phosphoglycerate. The wheat endosperm enzyme was found to be inhibited by orthophosphate, with 700 µM orthophosphate required to achieve half-maximal inhibition of enzyme activity. This is much more than the concentration required to achieve half-maximal inhibition of wheat leaf ADP-glucose pyrophosphorylase (56 µM). A similar lack of allosteric responsiveness of maize endosperm ADP-glucose pyrophosphorylase has been observed previously, In maize endosperm, this phenomenon has since been attributed to the effect of protease activity on the enzyme, since it has been demonstrated that maize endosperm ADP-glucose pyrophosphorylase is activated in vitro by 3-phosphoglycerate, when purified in the presence of 1.5 mM phenyl methane sulfonyl fluoride and 10 µg/ml chymostatin. However, we have purified the wheat endosperm ADP-glucose pyrophosphorylase enzyme in the presence of the protease inhibitors chymostatin, leupeptin and phenyl methane sulfonyl fluoride. We have been unable to demonstrate allosteric activation of this enzyme preparation in vitro by 3-phosphoglycerate. Thus, the results suggest that the wheat endosperm enzyme is not allosterically activated by 3-phosphoglycerate.

Consistent with the combined effect of 3-phosphoglycerate and orthophosphate on plant leaf ADP-glucose pyrophosphorylase enzymes, inhibition of the wheat endosperm enzyme is relieved by 3-phosphoglycerate. In the presence of 1 mM 3-phospho- glycerate, the I_{0.5} value of wheat endosperm ADP-glucose pyrophosphorylase for orthophosphate, is increased to 1.5 mM. This indicates that although the enzyme cannot undergo the necessary conformational changes necessary to convert it to a more active form in the presence of 3-phosphoglycerate, it is able to bind the metabolite. In view of this interaction of 3-phosphoglycerate and orthophosphate, the binding site for 3-phosphoglycerate is possibly close to the site for orthophosphate binding in the 3-dimensional structure of the enzyme.

### 1.3 Generation of antibodies to the enzyme

Sufficient protein to enable the immunisation of rabbits was obtained by pooling the isolated enzyme subunit isolated as described above from a number of separate experiments. The 51 kD ADP-glucose pyrophosphorylase polypeptide was subsequently purified to apparent homogeneity by electroelution from polyacrylamide gel slices. Antisera were prepared against the 51 kD ADP-glucose pyrophosphorylase polypeptide. The immunisation of rabbits was carried out essentially according to Mayer and Walker (1978).

The immune sera obtained precipitated ADP-glucose pyrophosphorylase activity from crude extracts of wheat endosperm. Following incubation of extracts with immune serum and centrifugation of the enzyme-Igg -protein A-Sepharose conjugates, only 10% of the ADP-glucose pyrophosphorylase activity remained in the supernatant fraction. The ADP-glucose pyrophosphorylase activity was detectable in the pellet fractions when the washed enzyme-Igg -protein A-Sepharose conjugates were assayed directly for enzyme activity. Approximately 70% of the total enzyme activity present in the endosperm extracts was subsequently recovered as an immunoprecipitate in pellet fractions. In control experiments, pre-immune sera collected from animals prior to their primary immunisation with ADP-glucose pyrophosphorylase did not partition ADP-glucose pyrophosphorylase activity. Western blot analysis of total wheat endosperm soluble protein using anti-wheat endosperm ADP-glucose pyrophosphorylase serum revealed the presence of a single 51 kD immunoreactive polypeptide. In a similar experiment, antibodies directed against the spinach leaf ADP-glucose pyrophosphorylase holoenzyme also recognised one polypeptide only in wheat endosperm of 51 kD molecular weight.

### 1.4 Subunit structure of endosperm and leaf enzymes

The presence of organ-specific isoenzymes of ADP-glucose pyrophosphorylase in wheat leaf and wheat endosperm was confirmed by western blot analysis of protein extracts from these organs, using anti-wheat endosperm ADP-glucose pyrophosphorylase serum to detect the enzyme subunits. As mentioned previously, the wheat endosperm enzyme consists of four 51 kD subunits. In western blotting experiments to determine the subunit structure of the wheat leaf isoenzyme, there was no detectable immunoreactive protein band in the crude extracts of wheat leaves. Following chromatography of wheat leaf extracts on phenyl-Sepharose and Superose 12 HR 10/30, a single immunoreactive polypeptide with a molecular weight of 48.5-49.0 kD was detected in western blots. The wheat leaf ADP-glucose pyrophosphorylase polypeptide is therefore approximately 2.5 kD smaller than the corresponding protein from wheat endosperm. Further evidence for the difference between the leaf and the endosperm enzymes is presented below.

### 2. ISOLATION AND CHARACTERISATION OF WHEAT ADP-GLUCOSE PYROPHOSPHORYLASE cDNAs

### 2.1 Identification of a wheat leaf ADP-glucose pyrophosphorylase cDNA clone

In order to identify a cDNA clone encoding wheat leaf ADP-glucose pyrophosphorylase, 3x10⁴ bacteriophage from the amplified wheat leaf cDNA library were screened with anti-spinach leaf ADP-glucose pyrophosphorylase serum. Of seven positive clones selected on the first screening, three clones producing the strongest signals were re-screened. Only one of these clones produced strong positive signals in subsequent rounds of screening. The intensity of the signal produced by this cDNA clone, relative to non-recombinant lgt11 suggests that the clone contains a cDNA encoding wheat leaf ADP-glucose pyrophosphorylase. The putative wheat leaf ADP-glucose pyrophosphorylase cDNA clone was designated WL:AGA.1. DNA was prepared from clone WL:AGA.1, Following restriction endonuclease digestion of the DNA with the enzyme EcoR1 and agarose gel electrophoresis, clone WL:AGA.1 was shown to contain a cDNA insert of 950 bp in size. The cDNA insert was labelled with ³²P by nick-translation and used to probe a northern blot of poly(A)-containing RNA from wheat leaf and wheat endosperm. This probe hybridises to mRNA bands of approximately 1.8 kb and 1.7 kb in size, respectively, in the endosperm and leaf RNA samples. The sizes of the wheat leaf and wheat endosperm ADP-glucose pyrophosphorylase mRNAs determined here are in close agreement with the estimates obtained by Krishnan et al (1986), from northern blots of wheat RNAs probed with a cDNA encoding rice endosperm ADP-glucose pyrophosphorylase. Comparison of the size of the cDNA insert with the size of the homologous mRNA species, indicates that the WL:AGA.1 cDNA insert contains approximately 55% of the complete wheat leaf ADP-glucose pyrophosphorylase mRNA sequence.

### 2.2 Sequence analysis of WL:AGA.1

The cDNA insert from clone WL:AGA.1 is 947 bp in length and encodes 301 amino acids at the C-terminus of wheat leaf ADP-glucose pyrophosphorylase. Approximately 70% of the mature wheat leaf ADP-glucose pyrophosphorylase polypeptide sequence is contained in the WL:AGA.1 protein. The cDNA extends 45 nucleotides into the 3'-untranslated region of the corresponding mRNA and terminates at the putative polyadenylation signal AATAAA ,located at positions 942-947. Consequently, there is no poly(A) tail in the wheat leaf ADP-glucose pyrophosphorylase cDNA sequence. The hydropathy index (Kyte and Doolittle, 1982) and secondary structure predictions (Chou and Fasman, 1978) were calculated for the WL:AGA.1 protein using the programmes of Devereux et al (1987). The Kyte and Doolittle (1982) hydropathy index of the WL:AGA.1 protein is consistent with its location in the soluble fraction of chloroplasts (Kaiser and Bassham. 1979b). The secondary structure predictions of Chou and Fasman (1978) have been used in comparisons of the wheat leaf and wheat endosperm ADP-glucose pyrophosphorylase proteins (see below).

### 2.3 Isolation of wheat endosperm ADP-glucose pyrophosphorylase cDNA clones

A wheat endosperm cDNA library was constructed. Double-stranded cDNA was prepared from oligo dT-cellulose-purified wheat endosperm RNA by a method employing RNaseH and E.coli DNA polymeraseI in the synthesis of the second strand, without prior purification of single-stranded cDNA (Gubler and Hoffman,1983). The unamplified wheat endosperm cDNA library was screened for the presence sequences homologous to the wheat leaf ADP-glucose pyrophosphorylase cDNA insert, WL:AGA.1. In a screen of 3x10⁴ recombinant bacteriophage, 10 positive signals were detected. Six of these clones were plaque-purified during two additional rounds of screening and designated WE:AGA.1, WE:AGA.3, WE:AGA.4, WE:AGA.5, WE:AGA.6 and WE:AGA.7. DNA was prepared from these six putative wheat endosperm ADP-glucose pyrophosphorylase cDNA clones. Following restriction endonuclease digestion of the DNAs with EcoR1 and agarose gel electrophoresis, the sizes of the cDNA inserts of these clones were shown to range from 400 bp to 1800 bp.

### 2.4 Sequence analysis of WE:AGA.3

The cDNA insert from clone WE:AGA.3 is 1272 bp in length, which includes a poly(A) tail 65 nucleotides long, at the 3'-terminus. The 3'-untranslated region of WE:AGA.3 cDNA, from the TAG stop codon to the start of the poly(A) tail, is 317 bp in length. There are two overlapping polyadenylation signals present in the 317 bp 3'untranslated region of WE:AGA.3 cDNA; the sequence AATAAA is located 104 bp upstream of the polyadenylation site, while the sequence AATAAG is 100 bp upstream of the polyadenylation site. Clone WE:AGA.3 also contains a third putative polyadenylation signal, AATAAA at position 1176-1181, 31 bp upstream from the polyadenylation site. Since the location of polyadenylation signals in plant mRNAs is usually 22-36 nucleotides upstream of the polyadenylation site (Joshi, 1987), then this third polyadenylation signal in clone WE:AGA.3 is the signal most likely to be involved with selection of the poly(A) addition site during processing of the corresponding ADP-glucose pyrophosphorylase mRNA. The open reading frame of clone WE:AGA.3 is 890 bp in length, encoding 296 amino acids of wheat endosperm ADP-glucose pyrophosphorylase. Thus, a protein of 33,200 molecular weight is encoded by clone WE:AGA.3, which corresponds to 65% of the size of mature wheat endosperm ADP-glucose pyrophosphorylase subunits.

### 2.5 Sequence analysis of WE:AGA.7

The largest wheat endosperm ADP-glucose pyrophosphorylase cDNA, WE:AGA.7 is 1798 bp in length comprising an open reading frame of 1500 bp. This open reading frame codes for a protein of 55,500 molecular weight, which exceeds the estimated molecular weight of the mature wheat endosperm ADP-glucose pyrophosphorylase polypeptide by approximately 4.5 kD. The amino acid sequence of the first 33 residues encoded by clone WE:AGA.7 exhibits properties similar to the transit peptides of chloroplast proteins (Colman and Robinson, 1986) and to the transit peptide of the amyloplast protein, granule-bound starch synthase (Klosgen et al, 1986). The sequence is rich in hydroxylated and basic amino acids, especially arginine which occurs every 4-5 residues in the sequence. Despite the similarity in amino acid content of the putative ADP-glucose pyrophosphorylase transit peptide and the granule-bound starch synthase transit peptide, there is no obvious sequence homology. There is also no sequence homology to the transit peptide sequences of nuclear-encoded chloroplast proteins, for example the small subunit of Rubisco (Mazur and Chui, 1985) and the 16 kD polypeptide of the oxygen-evolving complex (Jansen et al, 1987). The exact processing site for the pre-ADP-glucose pyrophosphorylase polypeptide has not been determined in these studies and will require N-terminal amino acid sequence analysis of the mature protein from wheat endosperm. However, cleavage of the precursor polypeptide between Met and Cys would produce a protein of 51,800, the approximate molecular weight of the mature ADP-glucose pyrophosphorylase. Furthermore, the chloroplast-specific transit peptide of the small subunit of Rubisco is also removed at the boundary between a cysteine and a methionine residue (Mazur and Chui, 1985), while the amyloplast-specific starch synthase transit peptide is removed at the boundary between a cysteine and an alanine residue (Klosgen et al.,1986).

In addition, clone WE:AGA.7 contains a 278 bp 3'-untranslated region, from the TAG stop codon to the start of the poly(A) tail. The untranslated region contains two overlapping putative polyadenylation signals, AATAAA at position 1728-1733 and AATAAG at position 1732-1737. These polyadenylation signals are located 45 bp and 41 bp respectively, from the polyadenylation site and are the same sequences present in clone WE:AGA.3. It is not possible at present to determine which of these signals may be functional. The Kyte and Doolittle (1982) hydropathy index and the Chou and Fasman (1978) secondary structure predictions were calculated for the WE:AGA.7 using the programme of Devereux et al (1987). The Kyte and Doolittle (1982) hydropathy profile indicates that the WE:AGA.7 protein contains hydrophobic and hydrophilic domains interspersed throughout its length, consistent with it being a soluble protein.

### 3. COMPARISON OF ADP-GLUCOSE PYROPHOSPHORYLASE SEQUENCES

### 3.1 Comparison of WG:AGA.3 and WG:AGA.7

The wheat endosperm ADP-glucose pyrophosphorylase cDNA inserts of clones WE:AGA.3 and WE:AGA.7 are 96.3% homologous in the shared regions of their open reading frames. In the 3'-untranslated region of these clones, from the TAG stop codons to the polyadenylation site, the extent of homology is reduced to 72.3%. A detailed comparison of the nucleotide sequences of WE:AGA.3 and WE:AGA.7 cDNAs reveals a total of 30 base substitutions, of which 72% are transitions. Within the open reading frame there are only 18 base substitutions, 11 transitions and 7 transversions. It is noteworthy that all of the transitions are located in third base positions of codons and none of them leads to an amino acid substitution. One of the transversions, located at position 1296 in WE:AGA.7 is silent. The remaining six transversions produce three conservative (threonine-serine, glutamine-lysine, alanine-serine) and two semi-conservative (arginine-methioine, isoleucine-methionine) amino acid substitutions. In addition, there are only 9 insertions/deletions in the open reading frame, occurring at positions 579-585 and positions 592-593 of the WE:AGA.3 nucleotide sequence. These insertions/deletions produce an additional 5 amino acid changes between WE:AGA.3 and WE:AGA.7 proteins, three insertions and 2 substitutions. Thus, the derived amino acid sequences of the wheat endosperm ADP-glucose pyrophosphorylase cDNAs differ in only 10 of 296 amino acid residues, yielding an amino acid homology of 96.7%. In the 3'-untranslated region there are a total of 85 insertions/deletions between WE:AGA.3 and WE:AGA.7 sequences, clustered into 8 variable regions.

### 3.2 Comparison of WE.AGA.7 and WL.AGA.1

The wheat leaf ADP-glucose pyrophosphorylase cDNA sequence (WL:AGA.1) and the wheat endosperm ADP-glucose pyrophosphorylase cDNA sequence (WE:AGA.7) were compared using the DIAGON programme of Devereux et al (1987). There are 10 well-conserved domains between the two nucleotide sequences. Between WL:AGA.1 and WE:AGA.7, there are 322 nucleotide substitutions, 290 of which are within the open reading frame, plus 148 insertions/deletions. Thus, within the open reading frames for which the WL:AGA.1 and WE:AGA.7 cDNA sequences overlap, there is only 55.7% homology at the DNA level. There is no homology in the 3'-untranslated region represented in the clones.

To determine the homology between WE:AGA.7 and WL:AGA.1 encoded polypeptides, the derived amino acid sequences were aligned. Of the 290 nucleotide substitutions within the open reading frame, 97 are silent. There are a total of 110 amino acid alterations between the wheat leaf and wheat endosperm ADP-glucose pyrophosphorylase polypeptide sequences, produced by the remaining 193 nucleotide substitutions and 91 of the 148 insertions/deletions. This data indicates that, in most Cases nucleotide substitutions between WL:AGA.1 and WE:AGA.7 cDNA sequences involve all three nucleotide positions of codons. Thus, there is only 55.3% homology between the derived amino acid sequences of these cDNAs.

Of the 110 amino acid substitutions between wheat leaf and wheat endosperm ADP-glucose pyrophosphorylase polypeptide sequences, 62 are conservative changes involving either no charge difference or substitution of charged residues for neutral amides (i.e. glutamine, asparagine), substitution of amphipathic residues for other amphipathic residues, or substitution of hydrophobic residues for other hydrophobic residues. There are 39 semi-conservative amino acid substitutions, involving reversal of charge, or substitution of amphipathic amino acids for charged, neutral, or hydrophobic residues. Non-conservative amino acid substitutions, of which there are 9, involve substitution of hydrophobic residues for either charged or neutral amino acids. Two of the non-conservative amino acid substitutions (His₅₁₂ -Tyr₅₁₂; Lys₅₂₁ -Gln₅₂₁) and two semi-conservative substitutions (Ser₅₀₈ -Glu₅₀₈ ; Ala₅₂₇ -Lys₅₂₇ ) between wheat leaf and wheat endosperm ADP-glucose pyrophosphorylase polypeptides are located in close proximity to, or within the region homologous to the putative 3-phosphoglycerate binding site on spinach leaf ADP-glucose pyrophosphorylase (Preiss et al, 1988). However, within the C-terminal 27 residues of wheat leaf and wheat endosperm ADP-glucose pyrophosphorylase enzymes the Chou and Fasman (1978) predictions are nearly identical.

### 3.3 Comparison of clones by restriction mapping

Restriction maps of the wheat leaf and wheat endosperm ADP-glucose pyrophosphorylase cDNAs were constructed to demonstrate their relatedness. The restriction map of the wheat leaf ADP-glucose pyrophosphorylase cDNA is very different from the maps of wheat endosperm ADP-glucose pyrophosphorylase cDNAs, with no internal restriction enzyme sites in common. This result confirms previous hybridisation analysis, which indicates that the wheat leaf and wheat endosperm ADP-glucose pyrophosphorylase genes cDNAs represent separate gene sub-families.In contrast, the wheat endosperm cDNAs WE:AGA.1, WE:AGA.3, WE:AGA.5, WE:AGA.6 and WE:AGA.7 are closely related. The cDNAs all contain the unique HindIII site located at the 3'-terminus, and the SstI site located at position -680 relative to the HindIII site. A unique BamHI site is present in the 5'-region of the longer cDNA inserts, WE:AGA.1, WE:AGA.6 and WE:AGA.7. There is polymorphism around the SstI site located at position -400 relative to the HindIII site of the wheat endosperm ADP-glucose pyrophosphorylase cDNAs, since this site is present only in clones WE:AGA.1, WE:AGA.6 and WE:AGA.7. In addition, the BglII site located at position -340 relative to the HindIII site, is only present in clones WE:AGA.3 and WE:AGA.5. These results suggest that the wheat endosperm ADP-glucose pyrophosphorylase gene sub-family may be divided into at least two distinct gene classes, with Class I represented by clones WE:AGA.3, WE:AGA.5 and Class II represented by clones WE:AGA.1, WE:AGA.6 and WE:AGA.7.

### 3.4 Comparison to other ADP-glucose pyrophosphorylase polypentides

The amino acid sequences derived from the wheat ADP-glucose pyrophosphorylase cDNAs were aligned with the amino acid sequences derived from a rice endosperm ADP-glucose pyrophosphorylase cDNA (Preiss et al, 1987) and the E.coli glgC gene (Baecker et al, 1983). The 5-way protein sequence alignment have been carried out. The homologies between amino acid sequences were subsequently calculated on the basis of this alignment as the proportion of identical residues in the overlapping regions and do not therefore take into account the size of gaps between the overlaps. There is 40% homology between the wheat endosperm ADP-glucose pyrophosphorylase sequences (WE:AGA.3 and WE:AGA.7) and rice endosperm ADP-glucose pyrophosphorylase. The wheat leaf sequence is 44% homologous to rice endosperm ADP-glucose pyrophosphorylase. These homologies are only slightly less than the 55% homology between wheat leaf and wheat endosperm sequences. In addition, there is 29.5% homology between the rice endosperm and E.coli amino acid sequences, compared to only 24% homology between the wheat leaf or wheat endosperm ADP-glucose pyrophosphorylases and E.coli ADP-glucose pyrophosphorylase.

### 3.5 Functional protein domains of ADP-glucose pyrophosphorylases

Regions of the E.coli and spinach leaf ADP-glucose pyrophosphorylase polypeptide sequences have been identified previously as substrate, activator or inhibitor binding sites (Parsons and Preiss, 1978a, 1978b; Larsen et al, 1986; Lee and Preiss, 1986; J. Preiss, personal communications). The derived amino acid sequences of wheat leaf and wheat endosperm ADP-glucose pyrophosphorylases have been compared with substrate, activator and inhibitor binding sites identified on other ADP-glucose pyrophosphorylase enzymes. These binding sites form five protein domains.
1. The fructose-1,6-bisphosphate binding site:
   The allosteric activator (fructose-1,6-bisphosphate) binding site of E.coli ADP-glucose pyrophosphorylase is located near the N-terminus of the protein close to the Lys₉₀ residue (Parsons and Preiss, 1978b). The amino acid sequence in wheat endosperm ADP-glucose pyrophosphorylase homologous to the fructose-1,6- bisphosphate binding site of the E.coli enzyme (Parsons and Preiss, 1978b) has been identified. The region from residue 78-100 has 14 out of the 23 amino acids conserved between wheat endosperm and E.coli sequences. However, there are major alterations at several amino acid positions, involving the substitution of charged residues in the E.coli sequence for hydrophobic or amphipathic residues in the wheat endosperm sequence , for example Arg₈₀ -Thr₈₀ , Lys₈₅ -Phe₈₅, Asp₈₆ -Pro₈₆ , Lys₉₀ -Thr₉₀ , Lys₉₃ -Thr₉₃ , His₉₇ -Pro₉₇ . Since pyridoxal-5'-phosphate binds at Lys90 in E.coli ADP-glucose pyrophosphorylase, and fructose-1,6-bisphosphate binds in this region also (Parsons and Preiss, 1978a, 1978b), this binding site is probably not functional in the wheat endosperm protein. Thus, in the wheat endosperm sequence homologous to the allosteric site of E.coli ADP-glucose pyrophosphorylase, there is no opportunity for Schiff base formation between fructose-1,6-bisphosphate and the protein domain. This finding is consistent with the lack of detectable activation of wheat endosperm ADP-glucose pyrophosphorylase by fructose-1,6-bisphosphate. Unfortunately, the wheat leaf ADP-glucose pyrophosphorylase cDNA sequence does not extend far enough into the 5'-coding region to contain sequences of the fructose-1,6-bisphosphate binding site. We cannot be certain, therefore, whether allosteric activation of leaf ADP-glucose pyrophosphorylases by fructose-1,6-bisphosphate is achieved by binding of the activators to the same site as in the E.coli enzyme.
2. Substrate binding sites: Two substrate binding sites of the E.coli ADP-glucose pyrophosphorylase are well-conserved in the wheat leaf and wheat endosperm ADP-glucose pyrophosphorylase sequences. The region between residues 166-173 contains a consensus sequence of WXXGTADA, obtained from a comparison of wheat endosperm, rice endosperm and E.coli protein sequences in this region. In all cases amino acid position 167 is occupied by an aromatic hydrophobic residue, either tyrosine or phenylalanine. Comparison of ADP-glucose pyrophosphorylases reveals a consensus sequence of FXEXP for residues 252-256. In addition, binding of pyridoxal-5'- phosphate to Lys₂₅₅ of the E.coli ADP-glucose pyrophosphorylase is prevented by ADP-glucose indicating that the substrate binding site is nearby. Although pyridoxal-5'-phosphate forms a Schiffs base with e-amino group of lysine, it is unlikely that Lys₂₅₅ is itself involved in substrate binding since it is not strictly conserved in the plant ADP-glucose pyrophosphorylase sequences: Clone WE:AGA.7, for example encodes a glutamine residue at position 255. However, Phe₂₅₁ is strictly conserved in all of the ADP-glucose pyrophosphorylase sequences examined, indicating that this residue might be involved in binding ATP and ADP-glucose, by hydrophobic interaction between the planar aromatic ring of the phenylalanine side-chain and the adenine ring of the substrate molecules. Nevertheless, an alternative mechanism involving ionic interaction between the phosphate moiety of ATP or ADP-glucose and the amino group of Arg₂₅₇ (or Lys₂₅₇) cannot be ruled out.
   In the putative substrate binding domains of ADP-glucose pyrophosphorylase enzymes there is no amino acid homology with the consensus sequence of Higgins et al (1986) for ATP binding sites. That consensus sequence (GXXXXGKS) has been shown by crystallographic analysis to form the phosphate binding region (Pai et al, 1979, cited in Higgins et al, 1986). A variant of this sequence, GDQLAEGKV in the wheat leaf sequence and SRLMSEGKV in the wheat endosperm sequences is located at position 460-468.
4. The 3-phosphoglycerate binding site: Amino acid sequences were compared to amino acid sequence of the putative 3-phosphoglycerate binding site on spinach leaf ADP-glucose pyrophosphorylase. This site is highly conserved between wheat leaf and wheat endosperm sequences. Different activation kinetics of the wheat leaf and wheat endosperm ADP-glucose pyrophosphorylase enzymes are not explained in terms of altered binding sites for 3-phosphoglycerate, since there is no obvious correlation between enzyme activation and conservation of the 3-phosphoglycerate binding site. In addition, although the primary sequences of wheat endosperm and wheat leaf ADP-glucose pyrophosphorylases differ in 6 of the 13 residues of the 3-phosphoglycerate binding site, the secondary structure predictions for this sequence are nearly identical.

Since 3-phosphoglycerate is able to prevent the inhibition of the wheat endosperm ADP-glucose pyrophosphorylase by orthophosphate this suggests that the enzyme possesses a functional 3-phosphoglycerate binding site. A consensus sequence for the binding of 3-phosphoglycerate, between residues 515-522 is SGIXXXXK, obtained by a comparison of all available plant ADP-glucose pyrophosphorylase sequences. Residue Lys₅₂₂ is strictly conserved in all sequences, and is involved with binding of pyridoxal-5'- phosphate, and probably also 3-phosphoglycerate, as found in the spinach leaf ADP-glucose pyrophosphorylase enzyme.

Thus, it is likely that alterations between wheat leaf and wheat endosperm ADP-glucose pyrophosphorylase amino acid sequences in regions other than the putative 3-phosphoglycerate binding site, are responsible for the observed differences in allosteric properties of these enzymes. Since the amino acid sequences of these two enzymes are only 55% homologous, any of the observed amino acid alterations might explain their different allosteric properties. This would suggest that the wheat endosperm enzyme might not be able to undergo the necessary conformational changes required to convert it to a more active form, in the presence of 3-phosphoglycerate.

## Claims

1. Plasmid WL:AGA.1 containing a cDNA encoding ADP-glucose pyrophosphorylase which plasmid has been deposited in an E.coli strain TG2 host with the National Collection of Industrial & Marine Bacteria, Aberdeen, U.K. on 19th October 1988: Accession No. NCIB 40065.

2. A cDNA encoding ADP-glucose pyrophosphorylase and having the same nucleotide sequence as that of the insert in plasmid WL:AGA.1 claimed in claim 1 or a sequence which is sufficiently similar thereto to to be capable of expressing a protein possessing ADP-glucose pyrophosphorylase activity.

3. Plasmid WE:AGA.3 containing a cDNA encoding ADP-glucose pyrophosphorylase which plasmid has been deposited in an E.coli strain TG2 host with the National Collection of Industrial & Marine Bacteria, Aberdeen, U.K. on 19th October 1988: Accession No. NCIB 40066.

4. A cDNA encoding ADP-glucose pyrophosphorylase and having the same nucleotide sequence as that of the insert in plasmid WL:AGA.3 claimed in claim 3 or a sequence which is sufficiently similar thereto to to be capable of expressing a protein possessing ADP-glucose pyrophosphorylase activity.

5. Plasmid WE:AGA.7 containing a cDNA encoding ADP-glucose pyrophosphorylase which plasmid has been deposited in an E.coli strain TG2 host with the National Collection of Industrial & Marine Bacteria, Aberdeen, U.K. on 19th October 1988: Accession No. NCIB 40067.

6. A cDNA encoding ADP-glucose pyrophosphorylase and having the same nucleotide sequence as that of the insert in plasmid WL:AGA.7 claimed in claim 5 or a sequence which is sufficiently similar thereto to to be capable of expressing a protein possessing ADP-glucose pyrophosphorylase activity.

7. A method of detecting the presence of a DNA sequence encoding ADP-glucose pyrophosphorylase comprising detecting a DNA digest with a gene probe comprising a cDNA as claimed in any of claims 2, 4, and 6.

8. A plant having enhanced ability to produce starch comprising a starch-synthesising plant having stably incorporated within its genome by transformation one or more than one additional copy of a gene encoding ADP-glucose pyrophosphorylase.

9. A plant having reduced ability to produce starch comprising a starch-synthesising plant having stably incorporated within its genome by transformation a gene encoding a mRNA antisense to the mRNA encoded by the endogenous ADP-glucose pyrophosphorylase gene of the plant.
